# EUROPEAN PATENT APPLICATION

(11) **EP 3 420 894 A1**
(43) Date of publication of application: **02.01.2019**
(21) Application number: 17178340.0
(22) Date of filing: 28.06.2017
(51) Int. Cl.: A61B 5/0215, A61B 5/027

(54) **INVASIVE MEDICAL DEVICE**

(71) Applicant: Koninklijke Philips N.V., 5656 AE Eindhoven (NL)
(72) Inventor: JOHNSON, Mark Thomas, 5656 AE Eindhoven (NL); HENDRIKS, Cornelis Petrus, 5656 AE Eindhoven (NL); HAKKENS, Franciscus Johannes Gerardus, 5656 AE Eindhoven (NL); LUCASSEN, Gerhardus Wilhelmus, 5656 AE Eindhoven (NL); HILGERS, Achim, 5656 AE Eindhoven (NL); VAN DEN ENDE, Daan Anton, 5656 AE Eindhoven (NL)
(74) Representative: de Haan, Poul Erik

(57) **Abstract**

An invasive medical device (10) is disclosed comprising a device portion (15) for inserting into a patient, said device portion including a functional module (20); an electrically conductive path (40) extending through the invasive medical device and connecting to the functional module; and a deformable switch (30) within the device portion arranged to alter the electrical impedance of said conductive path as a function of a degree of deformation of the switch, said deformation being caused by a blood parameter of the patient's blood that varies during the cardiac cycle of said patient.

## Description

### FIELD OF THE INVENTION

The present invention relates to an invasive medical device comprising a device portion for inserting into a patient, said device portion including a functional module and an electrically conductive path extending through the invasive medical device connected to the functional module.

### BACKGROUND OF THE INVENTION

Invasive medical devices, i.e. in-body devices such as catheters, guide wires or implantable medical devices can comprise one or more functional modules that are typically connected to a power source and optionally to a signal processor. In case of insertable invasive medical devices, such a functional module may be connected to the power source or the signal processor through one or more electrically conductive paths, e.g. wires or the like, that extend through the invasive medical device and connects the invasive medical device to a remote control unit including a power source and optionally including a signal processor, e.g. through one or more suitable connectors at a terminal end of the invasive medical device, whereas in case of an implantable medical device such conductive paths typically extend between a battery integral to the implantable medical device and the functional module and optionally between a signal processor integral to the implantable medical device and the functional module.

Such a functional module typically performs a function within the body of the patient in which the medical device is (partially) inserted. For example, the functional module may be an actuator that activates the release of a temporarily or permanently implantable object such as a valve, stent or the like from the invasive medical device acting as a delivery device, an ablation module at or near a distal end of an ablation catheter, an ultrasound or optical sensor for collecting in-body images of the patient, a steering member for changing the shape of the (tip of the) invasive medical device in order to guide or steer the invasive medical device to a desired location within the patient's body and so on.

Such invasive medical devices are typically inserted into a blood vessel such as an artery of the patient, where the invasive medical device is in direct contact with the patient's blood. Consequently, the invasive medical device is subjected to variations in certain blood parameters induced by the cardiac cycle of the patient, i.e. the repetitive expansion and contraction of the patient's heart in order to pump blood around the body of the patient. The variations in such blood parameters including blood pressure and resulting blood flow velocity can interfere with the intended operation of the invasive medical device.

For example, where the invasive medical device is a delivery device for delivering an implantable object to a desired location within the patient's body, variations in the blood flow velocity can cause movement of the implantable object away from its intended location, in particular during systolic blood pressure phases in which the blood flow velocity is highest.

Similarly, an ablation catheter needs to be kept in contact with the heart wall during ablation. However, as the heart wall is moving during the cardiac cycle, this leads to a variable contact force between the ablation catheter and the heart wall, which increases the risk of wall puncture where this contact force becomes too high.

Another example involves cardiac imaging techniques such as IVUS, ICE and OCT in which some of the images that are collected during the cardiac cycle are of limited use due to movement of structures within the heart such as the atrial wall during specific phases of the cardiac cycle.

Yet another example includes insertable medical devices such as catheters that can be deformed by a functional module, e.g. have a deformable tip at a distal end of the catheter, in order to steer the insertable medical device in an intended direction. An example of such a catheter is disclosed in US 2015/065953 A1, which includes an electromechanical polymer (EMP) actuator as its functional module disposed in a steerable tip at the distal end of the catheter. When activated, the EMP actuator deflects the steerable tip through an angle between 0 and 270 degrees, thus permitting the operator to steer the steerable tip through the vasculature. The steerable tip also has at least a first relatively stiff region and a second relatively flexible region, and the EMP actuator is provided next to the first relatively stiff region so that the steerable tip may move toward the flexible region when activated.

Variations in the blood flow velocity around such an insertable medical device can affect the degree of deformation of the insertable medical device, thereby hampering the desired steering of the insertable medical device and increasing the risk of accidental puncture of a blood vessel by the insertable medical device. This problem is addressed in US 2015/065953 A1 by the provision of one or more sensors that provide sensor signals representative of environment conditions surrounding the steerable tip. The sensor signals are relayed back to an external control circuit for processing. One or more EMP actuators acting as sensors may implement these sensors and may act as both actuator and sensor. The control circuit processes the sensor signals to adjust the electrical control signal dynamically. The sensors perform a pressure sensing function, and the deflection of the steerable tip is adjusted according to the sensed pressure to maintain a predetermined level of deflection.

This approach has the drawback that a feedback mechanism is required in order to operate the catheter in accordance with the sensed blood pressure. This therefore requires that sensor signals are routed back to the external control unit, which increases the number of electrically conductive paths, e.g. wires, in the catheter, thereby increasing the footprint of the catheter whilst further requiring such feedback signals to be processed.

### SUMMARY OF THE INVENTION

The present invention seeks to provide an invasive medical device, which operation can factor in variations in the aforementioned blood parameters as induced by the cardiac cycle without requiring a feedback mechanism.

According to an aspect, there is provided an invasive medical device comprising a device portion for inserting into a patient, said device portion including a functional module; an electrically conductive path extending through the invasive medical device and connecting to the functional module; and a deformable switch within the device portion arranged to alter the electrical impedance of said conductive path as a function of a degree of deformation of the switch, said deformation being caused by a blood parameter of the patient's blood that varies during the cardiac cycle of said patient. Hence, in accordance with the present invention no feedback mechanism is required to tune the operation of the functional module in accordance with the cardiac cycle-induced variations in the blood parameter due to the inclusion of the deformable switch in the electrically conductive path to the functional module. In embodiments of the present invention the electrically conductive path may be a power supply line although in alternative embodiments the electrically conductive path may be a signal line. The blood parameter may be blood pressure or blood flow velocity in preferred embodiments of the present invention.

In some embodiments, the functional module comprises an electroactive polymer and the electrically conductive path is arranged to supply an actuation signal to the electroactive polymer (EAP), e.g. an EMP, in which case the deformable switch may adjust or disrupt the actuation signal provided to the EAP as a function of the magnitude of the blood parameter, e.g. blood pressure or blood flow velocity. For example, the deformable switch may be arranged to reduce or increase the actuation signal with increasing blood pressure or blood flow velocity or alternatively may be arranged to disrupt or break the electrically conductive path upon a value of the blood parameter exceeding or falling below a defined threshold, e.g. exceeding or falling below a defined blood pressure value or a blood flow velocity value.

In an example embodiment, the deformable switch comprises a flexible membrane in an external surface of the device portion, said flexible membrane extending over a cavity filled with a compressible medium, said cavity comprising a cavity floor facing an inner surface of the flexible membrane, wherein said inner surface carries a first electrode arrangement and the cavity floor carries a second electrode arrangement facing the first electrode arrangement. Such an arrangement for example is particularly suitable for disrupting a power supply to the functional module at relatively low blood pressures where the flexible membrane is not sufficiently pushed into the cavity such that the first electrode arrangement is brought into physical contact with the second electrode arrangement in order to form the electrically conductive path to the functional module.

In other words, the electrically conductive path may be disrupted when the first electrode arrangement is spatially separated from the second electrode arrangement. However, it should be understood that alternative arrangements in which the conductivity of the electrically conductive path is a function of the distance between the first electrode arrangement and the second electrode arrangement. For example, the opposing electrode arrangements may form the plates of a capacitor or the like having a variable capacitance as a function of the distance between the two plates, which capacitor may be incorporated in an RC circuit having a variable RC time as a result of this variable capacitance, thereby giving the circuit including the switch a variable (pressure-sensitive) impedance.

In an example embodiment, the first electrode arrangement comprises a first electrode electrically connected to a first portion of the electrically conductive path and the second electrode arrangement comprises a second electrode electrically connected to a second portion of the electrically conductive path opposite said first portion. In this embodiment, both electrode arrangements are connected to a portion of the electrically conductive path. However, alternatively the first electrode arrangement comprises a first electrode and the second electrode arrangement comprises a pair of spatially separated further electrodes electrically connected to opposing portions of the electrically conductive path, wherein the first electrode is arranged to bridge the spatially separated further electrodes, i.e. to short the pair of further electrodes. This arrangement may be mirror-imaged such that the first electrode arrangement comprises a pair of spatially separated electrodes connected to opposing portions of the electrically conductive path and the second electrode arrangement on the cavity floor comprises the bridging electrode.

In yet another embodiment, the functional module forms part of a functional module arrangement implementing a plurality of functions including a first function enabled by the electrically conductive path and a second function enabled by a further electrically conductive path extending through the invasive medical device and connecting to the functional module arrangement, and wherein the deformable switch is arranged to disrupt the further electrically conductive path and enable the electrically conductive path at a first blood pressure of the patient and to disrupt the electrically conductive path and enable the further electrically conductive path at a second blood pressure of the patient different to said first blood pressure. This allows the functional module arrangement to switch between different functions, with each function being triggered when the cardiac cycle-dependent blood parameter, e.g. blood pressure or blood flow velocity, has a particular value.

In an alternative example embodiment, the deformable switch comprises a pressure-sensitive material portion in an external surface of the device portion, said pressure-sensitive material portion extending between opposing portions of the electrically conductive path and comprising a plurality of electrically conductive particles suspended in an electrically insulating matrix. Such a deformable switch is particularly suited for a gradual change in the electrical conductivity of the electrically conductive path by tuning the resistance of the deformable switch; upon compression of the deformable switch the conductive particles move closer together, thereby reducing the length of the resistive pathways in between the conductive particles and the overall resistance of the deformable switch as a consequence.

In embodiments in which the functional module comprises an EAP, the functional module may comprise a flexible module membrane for providing a balloon function, e.g. in an ablation catheter, wherein the electroactive polymer is arranged along an inner surface of the flexible module membrane. In this arrangement, with increasing blood pressure the EAP may become activated or more strongly activated such as to strengthen the resilience of the flexible module membrane against inward compression by the blood pressure, thereby preventing the balloon from partial collapse at increased blood pressures, e.g. systolic blood pressures.

Such an invasive medical device may further comprise a further electroactive polymer in between the flexible module membrane and the electroactive polymer arranged to control the shape of the flexible module membrane, wherein the electroactive polymer is arranged to support the further electroactive polymer as a function of the blood pressure of the patient.

In an alternative embodiment, the device portion is deformable, e.g. the deformable tip at a distal end of a catheter, and the electroactive polymer is arranged to deform said device portion, wherein the deformable switch is arranged to alter the electrical impedance of said conductive path with increasing deformation of the device portion. For example, at high blood flow velocities during systolic blood pressure, where the deformation of the device portion is counteracted by high blood flow velocities, the electrical conductivity of the path providing the EAP with its actuation signal may be reduced to a lesser extent or not at all compared to lower blood flow velocities during diastolic blood pressure during which the deformation of the device portion is counteracted to a lesser degree by the blood flow. In this manner, a relatively constant degree of deformation of the device portion can be maintained throughout the patient's cardiac cycle.

In this embodiment, the deformable switch may comprise a pair of conductive portions connected to opposing portions of the electrically conductive path, wherein the conductive portions are slidably arranged relative to each other such that a contact area between the conductive portions is altered with increasing deformation of the device portion. In this manner, the electrically conductive path for example may be disrupted upon the degree of deformation of the device portion reaching a critical value, thereby preventing the device portion from being deformed beyond this critical value, which may cause damage to the device portion and/or a blood vessel of the patient.

The invasive medical device may be a temporarily insertable medical device such as a catheter or like or alternatively the invasive medical device may be an implantable medical device comprising a battery, wherein the electrically conductive path extends between the battery and the functional module. This for example may help to extend the lifetime of such a battery by disabling the functional module during parts of the cardiac cycle of the patient where the functional module, e.g. a sensor or the like, cannot collect reliable or useful data.

The deformable switch may be separate to the functional module, e.g. spatially separated therefrom, or alternatively may be integral to the functional module, thereby obviating the need for a separate component in the invasive medical device.

### BRIEF DESCRIPTION OF THE DRAWINGS

Embodiments of the invention are described in more detail and by way of nonlimiting examples with reference to the accompanying drawings, wherein:
FIG. 1 schematically depicts a cross-sectional view of an invasive medical device according to an embodiment;
FIG. 2 schematically depicts a cross-sectional view of part of an invasive medical device and its operating principle according to an example embodiment;
FIG. 3 schematically depicts a cross-sectional view of part of an invasive medical device and its operating principle according to another example embodiment;
FIG. 4 schematically depicts a cross-sectional view of part of an invasive medical device and its operating principle according to yet another example embodiment; FIG. 5 schematically depicts a cross-sectional view of part of an invasive medical device and its operating principle according to still another example embodiment;
FIG. 6 depicts a graph of a typical aortic blood pressure during the cardiac cycle;
FIG. 7 schematically depicts a cross-sectional view of part of an invasive medical device according to a further example embodiment;
FIG. 8 schematically depicts a cross-sectional view of an aspect of an invasive medical device according to yet another embodiment;
FIG. 9 schematically depicts a cross-sectional view of part of an invasive medical device according to a further embodiment;
FIG. 10 schematically depicts a cross-sectional view of an invasive medical device according to an alternative embodiment;
FIG. 11 is a graph depicting blood flow velocities during the cardiac cycle;
FIG. 12 schematically depicts a deformable invasive medical device within a patient's blood vessel; and
FIG. 13 schematically depicts a cross-sectional view of a deformable invasive medical device according to an embodiment of the present invention.

### DETAILED DESCRIPTION OF THE EMBODIMENTS

It should be understood that the Figures are merely schematic and are not drawn to scale. It should also be understood that the same reference numerals are used throughout the Figures to indicate the same or similar parts.

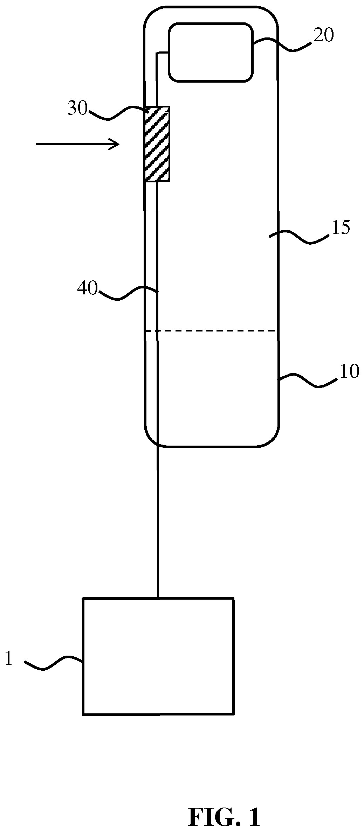

FIG. 1 schematically depicts an invasive medical device 10 in accordance with a first set of embodiments of the present invention. The invasive medical device 10 is a device for inserting into a patient, for example into the vascular system of the patient such that a device portion 15 of the invasive medical device 10 becomes inserted into the patient. For example, the invasive medical device 10 maybe a catheter, guide wire, implantable object delivery device or an implantable medical device although other suitable types of invasive medical devices will be immediately apparent to the skilled person.

The device portion 15 of the invasive medical device 10 comprises one or more functional modules 20 that form a functional module arrangement of the invasive medical device 10. In the context of the present application, a functional module 20 is a module that performs a function at the device portion 15 at least when the invasive medical device 10 is inserted into the patient. Such a function may be triggered through an electrically conductive path 40 extending through the invasive medical device 10 and connecting the functional module 22 an external control unit 1, e.g. a user interface or the like for operating the invasive medical device 10 when inserted into the patient.

The functional module 20 may perform any suitable function. For example, the functional module 20 maybe an actuator, such as an actuator for releasing an implantable object carried by the invasive medical device 10 into the patient's body, in which embodiments the invasive medical device 10 may be a catheter or object delivery device from which the implantable object is released upon actuation of the functional module 20 with an actuation signal provided to the functional module through the electrically conductive path 40. Such an implantable object maybe a stent or the like, for example, for implanting in a coronary artery of the patient. The actuator alternatively may be used to alter the shape of the device portion 15, e.g. in case of a deformable device portion 15 such as the tip at the distal end of a catheter. Alternatively, the functional module 20 maybe a sensor such as an ultrasound or optical sensor for collecting image data from within the patient's body, e.g. cardiac images. Other embodiments of such a functional module 20 will be apparent to the skilled person.

In some embodiments, the functional module 20 may comprise an EAP-based actuator or sensor. Electroactive polymers (EAPs) are an emerging class of materials within the field of electrically responsive materials. EAPs can work as sensors or actuators and can easily be manufactured into various shapes allowing easy integration into a large variety of systems. Materials have been developed with characteristics such as actuation stress and strain which have improved significantly over the last ten years. Technology risks have been reduced to acceptable levels for product development so that EAPs are commercially and technically becoming of increasing interest. Advantages of EAPs include low power, small form factor, flexibility, noiseless operation, accuracy, the possibility of high resolution, fast response times, and cyclic actuation. The improved performance and particular advantages of EAP materials give rise to applicability to new applications.

An EAP-based functional module 20 can be used in any application in which a small amount of movement of a component or feature is desired, based on electric actuation. Similarly, the technology can be used for sensing small movements.

The use of EAPs enables functions which were not possible before, or offers a big advantage over common sensor/actuator solutions, due to the combination of a relatively large deformation and force in a small volume or thin form factor, compared to common actuators. EAPs also give noiseless operation, accurate electronic control, fast response, and a large range of possible actuation frequencies, such as 0 - 1MHz, most typically below 20 kHz.

Devices using electroactive polymers can be subdivided into field-driven and ionic-driven materials.

Examples of field-driven EAPs include Piezoelectric polymers, Electrostrictive polymers (such as PVDF-based relaxor polymers) and Dielectric Elastomers. Other examples include Electrostrictive Graft polymers, Electrostrictive paper, Electrets, Electroviscoelastic Elastomers and Liquid Crystal Elastomers.

Examples of ionic-driven EAPs are conjugated/conducting polymers, Ionic Polymer Metal Composites (IPMC) and carbon nanotubes (CNTs). Other examples include ionic polymer gels.

In embodiments of the invention, the functional module 20 comprises at least one field-driven electroactive material actuator or sensor.

Field-driven EAPs are actuated by an electric field through direct electromechanical coupling. They usually require high fields (volts per meter) but low currents. Polymer layers are usually thin to keep the driving voltage as low as possible. Ionic EAPs are activated by an electrically induced transport of ions and/or solvent. They usually require low voltages but high currents. They require a liquid/gel electrolyte medium (although some material systems can also operate using solid electrolytes). Both classes of EAP have multiple family members, each having their own advantages and disadvantages.

A first notable subclass of field-driven EAPs includes Piezoelectric and Electrostrictive polymers. While the electromechanical performance of traditional piezoelectric polymers is limited, a breakthrough in improving this performance has led to PVDF relaxor polymers, which show spontaneous electric polarization (field-driven alignment). These materials can be pre-strained for improved performance in the strained direction (pre-strain leads to better molecular alignment). Normally, metal electrodes are used since strains usually are in the moderate regime (1-5%). Other types of electrodes (such as conducting polymers, carbon black based oils, gels or elastomers, etc.) can also be used. The electrodes can be continuous, or segmented.

Another subclass of interest of field-driven EAPs is that of Dielectric Elastomers. A thin film of this material maybe sandwiched between compliant electrodes, forming a parallel plate capacitor. In the case of dielectric elastomers, the Maxwell stress induced by the applied electric field results in a stress on the film, causing it to contract in thickness and expand in area. Strain performance is typically enlarged by pre-straining the elastomer (requiring a frame to hold the pre-strain). Strains can be considerable (10-300%). This also constrains the type of electrodes that can be used: for low and moderate strains, metal electrodes and conducting polymer electrodes can be considered, for the high-strain regime, carbon black based oils, gels or elastomers are typically used. The electrodes can be continuous, or segmented.

In some cases, thin film electrodes may be added when the polymer itself lacks sufficient conductivity (dimension-wise). The electrolyte can be a liquid, a gel or a solid material (i.e. complex of high molecular weight polymers and metal salts). Most common conjugated polymers are polypyrrole (PPy), Polyaniline (PANi) and polythiophene (PTh).

An actuator may also be formed of carbon nanotubes (CNTs), suspended in an electrolyte. The electrolyte forms a double layer with the nanotubes, allowing injection of charges. This double-layer charge injection is considered as the primary mechanism in CNT actuators. The CNT acts as an electrode capacitor with charge injected into the CNT, which is then balanced by an electrical double-layer formed by movement of electrolytes to the CNT surface. Changing the charge on the carbon atoms results in changes of C-C bond length. As a result, expansion and contraction of single CNT can be observed.

In accordance with embodiments of the present invention, the invasive medical device 10 further comprises a deformable switch 30 arranged to alter the electrical impedance of the electrically conductive path 40 as a function of a change in a blood parameter induced by a particular phase of the cardiac cycle of the patient. In the context of the present application, such a change in electrical impedance of the electrically conductive path 40 includes a change in the electrical conductivity of the electrically conductive path 40, e.g. a disruption or establishment of the electrically conductive path 40 although embodiments of the present invention are not limited thereto, as will be explained in more detail below. In such embodiments, the electrically conductive path 40 may form part of a power supply arrangement to the functional module 20, which power supply arrangement may be realized in any suitable manner, as is well-known per se to the skilled person and will therefore not be explained in further detail for the sake of brevity only.

In FIG. 1, the deformable switch 30 is a blood pressure-sensitive switch 30 that exhibits a degree of deformation that is a function of the blood pressure exerted onto the deformable switch 30. For example, the degree of deformation of the deformable switch 30 may increase with increasing blood pressure of the patient.

As will be explained in further detail below, this deformation may be used to alter the electrical conductivity of the electrically conductive path 40. In a first set of embodiments, the deformable switch 30 may switch between a first state in which the electrically conductive path 40 is disrupted and a second state in which the electrically conductive path 40 is conductive. In a second set of embodiments, the deformable switch 30 may alter the resistance or impedance in a continuous manner as a function of its degree of deformation. The electrically conductive path 40 may be realized in any suitable manner, e.g. as a conductive track, wire, cable, and so on.

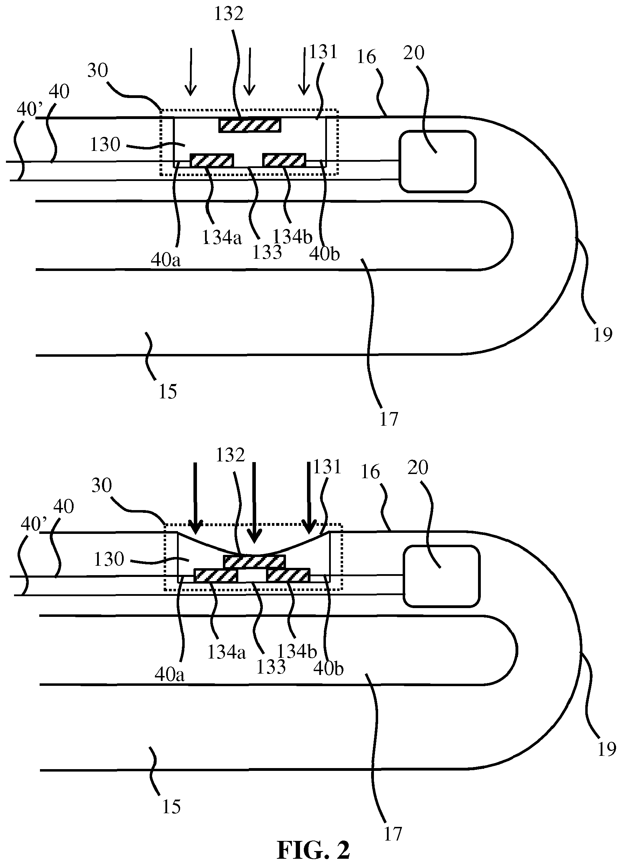

FIG. 2 schematically depicts an example embodiment of a device portion 15 of an invasive medical device 10 in which the deformable switch 30 is formed in or on an external surface 16 of the device portion 15. In this embodiment, the deformable switch 30 comprises a flexible membrane 131 spanning across a cavity 130 in the device portion 15, which cavity 130 is filled with a compressible medium such as air. The flexible membrane may be made of any suitable flexible material, e.g. a flexible polymer such as a biocompatible elastomer, e.g. rubber.

An inner surface of the flexible membrane 131 facing the cavity 130 carries a first electrode arrangement here including a first electrode 132. The cavity 130 comprises a cavity floor 133 opposite the inner surface of the flexible membrane 131 carrying a second electrode arrangement, here comprising a pair of spatially separated electrodes 134a, 134b, with the electrode 134a being connected to a first portion 40a and the electrode 134b being connected to a second portion 40b of the electrically conductive path 40.

The respective electrodes of the first electrode arrangement and the second electrode arrangement individually may be made of any suitable electrically conductive material, e.g. a metal, metal alloy, conductive polymer, conductive composite material, and so on.

The electrically conductive path 40 may be a power supply line to the functional module 20 or alternatively may be a signal line of the functional module 20. The functional module 20 optionally may be connected to one or more additional electrically conductive paths 40', which for example may be at least one of a further power supply line, e.g. a ground line, and a further signal line.

The invasive medical device 10 in some embodiments may be a catheter, which catheter optionally may comprise a hollow channel 17 through which an instrument or guide wire may be guided through the tip 19 of the catheter as is well-known per se, such that this will not be explained in further detail for the sake of brevity only.

The operation of the deformable switch 30 as depicted in FIG. 2 is as follows. As shown in the top pane by the arrows towards the flexible membrane 131, a relatively low blood pressure such as a diastolic blood pressure does not significantly deform the flexible membrane 131 such that the electrode 132 of the first electrode arrangement is spatially separated from the electrodes 134a, 134b of the second electrode arrangement on the cavity floor 133 and the electrically conductive path 40 is disrupted as a consequence. However, at increasing blood pressure as schematically depicted in the lower pane of FIG. 2 by the larger arrows towards the flexible membrane 131, e.g. systolic blood pressure, the flexible membrane 131 is deformed (pushed) into the cavity 130 such that the electrode 132 of the first electrode arrangement carried by the flexible membrane 131 bridges the spatially separated electrodes 134a, 134b of the second electrode arrangement on the cavity floor 133, thereby electrically connecting the first portion 40a to the second portion 40b of the electrically conductive path 40 such that a power supply or a signal may be communicated between the external control unit 1 and the functional module 20. Hence, in this embodiment the deformable switch 30 disrupts the electrically conductive path 40 upon the blood pressure of the patient dropping below a certain threshold pressure at which the first electrode arrangement on the flexible membrane 131 no longer contacts the second electrode arrangement on the cavity floor 133.

In the above embodiment, the first electrode arrangement is not connected to the electrically conductive path 40 but acts as a bridging electrode for the second electrode arrangement as explained above. It will be understood that although not specifically shown the mirror image arrangement in which the first electrode arrangement comprises a pair of spatially separated electrodes connected to opposing portions of the electrically conductive path 40 and the second electrode arrangement comprises the bridging electrode is of course equally feasible.

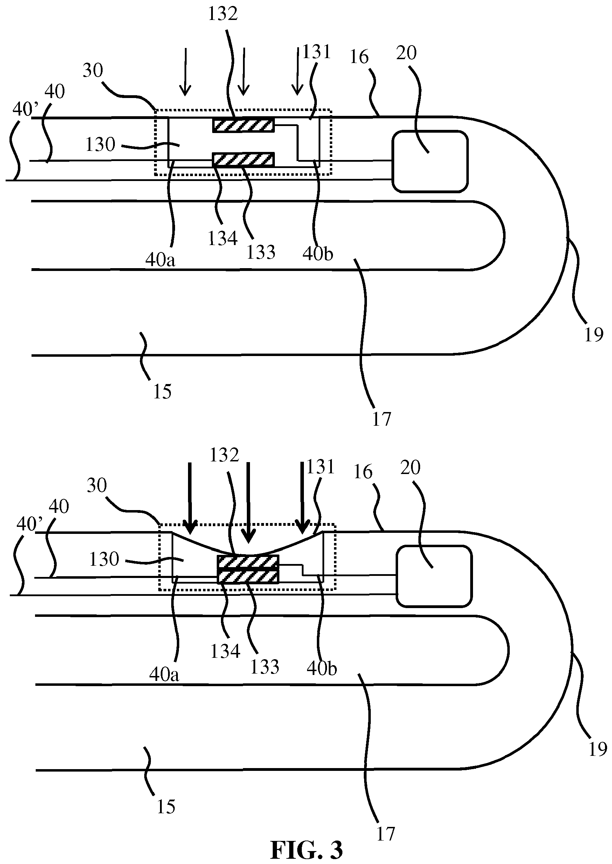

FIG. 3 schematically depicts another example embodiment of a device portion 15 of an invasive medical device 10 in which the deformable switch 30 is formed in or on an external surface 16 of the device portion 15 and comprises a flexible membrane 131 spanning a cavity 130 in the external surface 16. In this embodiment, the first electrode arrangement on the inner surface of the flexible membrane 131 facing the cavity floor 133 comprises a first electrode 132 connected to a first portion of the electrically conductive path 40, e.g. the portion 40b, whereas the second electrode arrangement on the cavity floor 133 comprises a second electrode 134 opposing the first electrode 132, with the second electrode 134 being connected to an opposing portion of the electrically conductive path 40, e.g. the portion 40a. Of course, the first electrode 132 may be connected to the portion 40a and the second electrode 134 may be connected to the portion 40b instead.

As with the deformable switch 30 in the example embodiment schematically depicted in FIG. 2, the deformable switch 30 in the example embodiment schematically depicted in FIG. 3 disrupts the electrically conductive path 40 at low blood pressure, e.g. diastolic blood pressure, as schematically depicted in the top pane of FIG. 3 due to the spatial separation between the first electrode 132 and the second electrode 134, whereas increased deformation of the flexible membrane 131 at increasing blood pressure of the patient, e.g. systolic blood pressure, causes the first electrode 132 to contact the second electrode 134, thereby restoring or enabling the electrically conductive path 40.

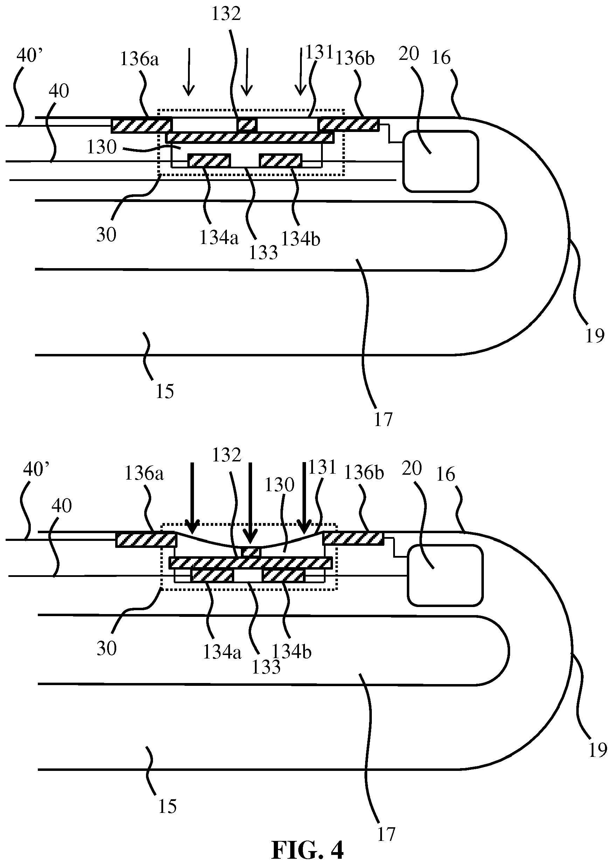

FIG. 4 schematically depicts another example embodiment of a device portion 15 of an invasive medical device 10 in which the deformable switch 30 is formed in or on an external surface 16 of the device portion 15 and comprises a flexible membrane 131 spanning a cavity 130 in the external surface 16. In this embodiment, the deformable switch 30 implements multiple states, i.e. more states than the on/off states of the functional module 20 in the example embodiments schematically depicted in FIG. 2 and FIG. 3. In this embodiment, the deformable switch may comprise a third electrode arrangement comprising spatially separated electrodes 136a and 136b connected to opposing portions of a further electrically conductive path 40' in addition to the first electrode arrangement on the inner surface of the flexible membrane 130, here a bridging electrode 132, and the second electrode arrangement on the cavity floor 133, here comprising a pair of spatially separated electrodes 134a, 134b connected to opposing portions of the electrically conductive path 40.

The third electrode arrangement including the spatially separated electrodes 136a, 136b may be arranged along the external surface 16 such that at a low blood pressure of the patient, e.g. diastolic blood pressure, as shown in the top pane of FIG. 4, the bridging electrode 132 bridges the spatially separated electrodes 136a, 136b, thereby enabling the electrically conductive path 40' whilst disrupting the electrically conductive path 40. In contrast, when the flexible membrane 131 is deformed into the cavity 130 at higher blood pressure of the patient, e.g. systolic blood pressure, as shown in the bottom pane of FIG. 4, the bridging electrode 132 bridges the spatially separated electrodes 134a, 134b of the second electrode arrangement, thereby enabling the electrically conductive path 40 whilst disrupting the electrically conductive path 40'. As will be readily understood by the skilled person, at intermediate blood pressures a third state may exist in which the bridging electrode 132 is spatially separated from the second as well as the third electrode arrangements such that in this state both the electrically conductive path 40 and the electrically conductive path 40' are disrupted.

Such a multi-state deformable switch 30 for example maybe utilized in an invasive medical device 10 comprising a functional module arrangement implementing multiple functions, e.g. a single functional module 20 implementing such multiple functions or multiple functional modules 20 each implementing a different function, wherein different functions may be enabled at different blood pressures of the patient. It is furthermore noted for the avoidance of doubt that such a multi-state deformable switch 30 may be implemented in any suitable manner. For example, such as switch 30 may be implemented using a three-terminal solution in which two feeding or input electrodes are connected to one common electrode or vice versa. In yet another example embodiment, such a multi-state deformable switch 30 may be used in combination with a functional module 20 comprising an EAP actuator in which the different states of the deformable switch 30 are used to define different actuation levels for the EAP actuator or alternatively the functional module 20 may comprise multiple EAP actuators where the different states of the deformable switch 30 actuate different EAP actuators.

At this point it is further noted that embodiments of the present invention are not limited to a single deformable switch 30 in a single electrically conductive path 40. It is equally feasible to include multiple deformable switches 30 within such an electrically conductive path 40, e.g. to tune the functionality of the functional module 20. Such multiple switches 30 within a single electrically conductive path 40 maybe any combination of switches 30 as described in the various example embodiments within the present application.

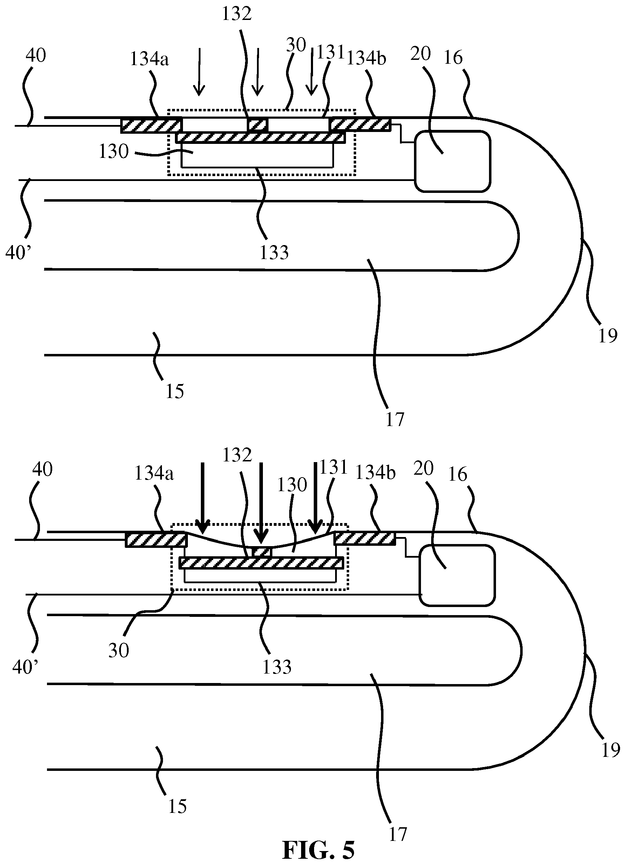

FIG. 5 schematically depicts another example embodiment of a device portion 15 of an invasive medical device 10 in which the deformable switch 30 is formed in or on an external surface 16 of the device portion 15 and comprises a flexible membrane 131 spanning a cavity 130 in the external surface 16. In this embodiment, the second electrode arrangement including spatially separated electrodes 134a and 134b connected to opposing portions of the electrically conductive path 40 is arranged along the external surface 16 of the device portion 15 such that the bridging electrode 132 of the first electrode arrangement on the inner surface of the flexible membrane 131 contacts the spatially separated electrodes 134a, 134b of the second electrode arrangement at low blood pressure, e.g. diastolic blood pressure, of the patient as shown in the top pane of FIG. 5.

However, when the flexible membrane 131 is pushed into the cavity 130 at increasing blood pressures, e.g. systolic blood pressure, the electrically conductive path 40 is disrupted as the bridging electrode 131 is spatially separated from the spatially separated electrodes 134a, 134b as shown in the bottom pane of FIG. 5. Hence, in this example embodiment the deformable switch 30 enables the electrically conductive path 40 at low blood pressure, e.g. diastolic blood pressure, which for example may be advantageous where the functional module 20 is an actuator for delivering an implantable object at an intended location, as such a delivery preferably takes place at low blood flow velocity is associated with low blood pressures to reduce the risk of the implantable object being displaced by the blood flow.

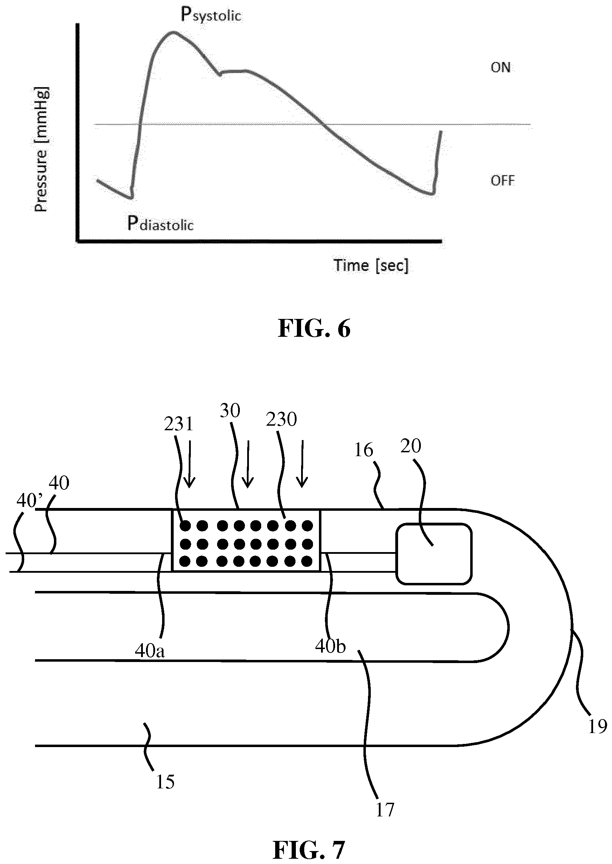

FIG. 6 depicts a graph of the aortic blood pressure during the cardiac cycle, including the systolic pressure P_{systolic} and the diastolic pressure P_{diastolic}. The horizontal line indicates a threshold pressure above which the deformable switch 30 may switch on the functional module 20 and below which the deformable switch 30 may switch off the functional module 20, by disrupting the electrically conductive path 40 as previously explained. Of course, as will be apparent from the foregoing in alternative embodiments the deformable switch 30 may switch on the functional module 20 at blood pressures below this threshold blood pressure and may switch off the functional module 20 at blood pressures above this threshold blood pressure.

In embodiments in which the deformable switch 30 is used to control an EAP actuator of the functional module 20, the deactivation time of such an actuator when disrupting the electrically conductive path 40 may depend on the internal resistance of the EAP actuator. In such embodiments, the deactivation time of such an actuator may be further tuned by the provision of a resistor in parallel with the deformable switch 30 that determines the deactivation time of the EAP actuator during disruption of the electrically conductive path 40.

At this point, it is noted that embodiments of the present invention are not limited to binary deformable switches 30, i.e. switches that switch between and 'on' and an 'off' state of the functional module 20. For example, in case of the flexible membrane 131-based deformable switches 30 described in more detail above, the deformable switch 30 may have a complex impedance represented by a variable capacitance or inductance. In this manner, an AC part of a driving voltage of the functional module 20 may also become a function of the blood pressure of the patient. This may be realized by the first electrode arrangement and the second electrode arrangement defining opposing plates of a capacitor in which case the capacitance of this capacitor becomes a function of the distance between the opposing plates of the capacitor as is well-known per se. Alternatively, a variable inductance may be achieved by the use of an inductive material such as a ferrite material suspended on the flexible membrane 131 over a meandering conductive track, i.e. the electrically conductive path 40, such that the inductance becomes a function of the distance between the inductive material and the conductive track.

As will be understood by the skilled person, the different types of deformable switches maybe combined, e.g. combinations of variable capacitive, inductive and/or resistive blood pressure sensitive switches 30 in parallel and/or series connections in order to tune the functionality of the functional module 20. This is particularly advantageous when the functional module 20 comprises one or more EAP actuators in order to tune the behaviour of the EAP actuators. For example, a combination of blood pressure-sensitive deformable switches 30 having different threshold levels as explained in FIG. 6 maybe used to control the degree of actuation, e.g. deformation, of such EAP actuators although it should be understood that embodiments of the present invention are not limited to invasive medical devices 10 comprising one or more functional modules 20 comprising EAP materials as previously explained.

In yet another example embodiment, the functional module 20 may be coupled to multiple power supply lines, i.e. electrically conductive paths 40, with the deformable switch 30 being arranged in one of these paths such that a fraction of the power supply to the functional module 20 is applied to the module as a function of the variable blood parameter, e.g. blood pressure.

Fig. 7 schematically depicts a device portion 15 of an invasive medical device 10 according to yet another embodiment. In this embodiment, the deformable switch 30 is inserted in the electrically conductive path 40 as a blood pressure-sensitive deformable switch 30 having a variable resistance as a function of the blood pressure. In this embodiment, the deformable switch 30 comprises a deformable matrix material 230 that is electrically insulating, e.g. a deformable polymer such as an elastomer or the like such as silicone, polyurethane, polyether block amide (PEBA), polybutadiene rubber, and so on, in which electrically conductive particles 231, e.g. metal, metal alloy, conductive metal oxide particles, carbon-based particles such as carbon black particles, graphite particles, graphene flakes, carbon nanotubes, and so on, are suspended. A portion of the deformable switch 30 is exposed at the external surface 16 of the device portion 15 of the invasive medical device 10 such that the deformable matrix material 230 can be deformed as a function of the blood pressure exerted on this exposed portion as indicated by the arrows directed at this portion. At increasing blood pressures, the deformable matrix material 230 is increasingly deformed, i.e. compressed, such that the conductive particles 231 are brought closer together, thereby reducing the resistance of the deformable switch 30. Consequently, the voltage drop across the deformable switch 30 is a function of the blood pressure exerted on the deformable switch 30. For example, where the functional module 20 comprises an EAP actuator operated with a DC operating voltage, the voltage drop and corresponding mechanical actuation of the EAP would change accordingly.

In a preferred embodiment, the concentration of the conductive particles 231 in the matrix 230 is close to the percolation level such that upon compression of the deformable switch 30, e.g. at systolic blood pressure, the conductive particles 231 contact each other and provide a conductive pathway between the opposing portions 40a, 40b of the electrically conductive path 40.

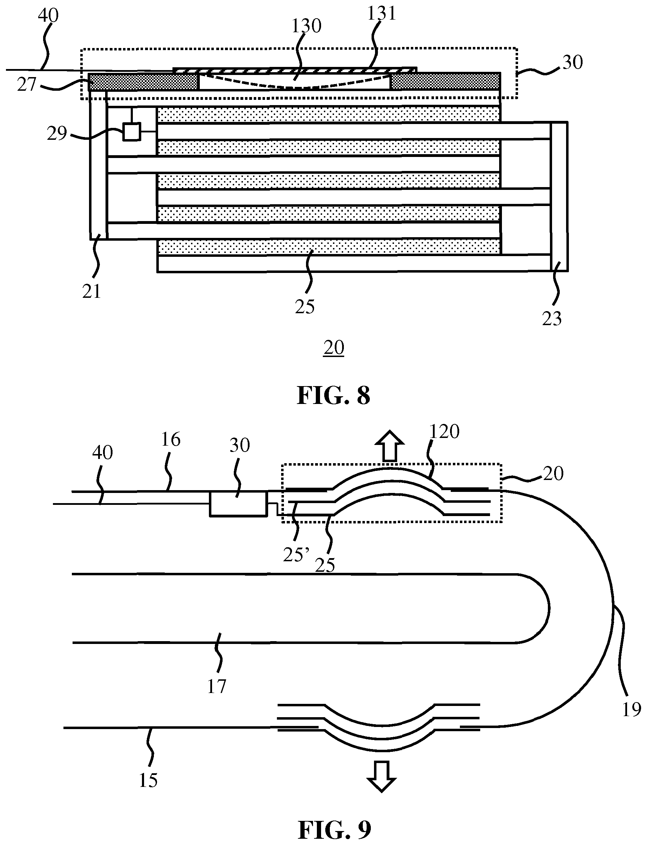

In the so far described embodiments, the deformable switch 30 is provided as a separate entity to the functional module 20 in the device portion 15 of the invasive medical device 10. However, in another example embodiment schematically depicted in FIG. 8, the deformable switch 30 forms part of, i.e. is integrated in, the functional module 20, here a multilayer EAP actuator comprising a plurality of EAP layers 25 sandwiched in between interdigitated electrodes 21 and 23. The deformable switch 30 is mounted on top of the multilayer stack and comprises a flexible membrane 131 forming a top electrode connected to the electrically conductive path 40 and suspended over a cavity 131. The top electrode formed by the flexible membrane 131 is electrically insulated from the electrode arrangement, e.g. the first electrode arrangement 21 by an electrically insulating material 27 in which the cavity 130 is formed. At increasing blood pressure of the patient, e.g. systolic blood pressure, the flexible membrane 131 deforms towards the first electrode arrangement 21 and contacts the portion of the first electrode arrangement 21 defining the cavity floor.

As previously explained, a parallel resistor or RC element 29 maybe included to tune the discharge time of the EAP actuator if the native RC time of the EAP actuator resulting from the disruption of the electrically conductive path 40 is sub-optimal. Again, it is noted that the integration of such a deformable switch 30 in a functional module 20 is not limited to EAP-based modules and may be integrated in any suitable type of actuator and/or sensor-type functional modules 20, e.g. to disrupt a contact between the actuator and the control unit 1 or to short such an actuator or sensor.

FIG. 9 schematically depicts yet another example embodiment of the present invention, in which the device portion 15 of an invasive medical device 10 is depicted, here an ablation catheter comprising a functional module 20 arranged to implement a balloon function around the external surface of the invasive medical device 10, e.g. at or near the tip at the distal end of the invasive medical device 10. Such a functional module 20 may comprise a flexible membrane 120 mounted in or on the device portion 15, which flexible membrane 120 maybe controlled by an actuator 25', e.g. an EAP actuator, in order to expand the flexible membrane 120 into a blood vessel or part of the heart of the patient, as indicated by the block arrows. The actuator 25' maybe actuated in any suitable manner, e.g. by an actuation signal generated with the control unit 1 and supplied over any suitable type of electrically conductive path (not shown).

A challenge with such ablation catheters or other balloon-type medical devices is that the blood pressure typically provides a counterforce on the flexible membrane 120 such that at higher blood pressures, e.g. systolic blood pressure, the actuator 25' has to deliver a greater force in order to maintain the desired expansion of the balloon. In other words, where such an actuator 25' delivers a constant force during the cardiac cycle, the shape of such a balloon will vary accordingly.

In order to address this problem, a supporting EAP layer 25 may be provided behind the actuator 25' such that the actuator 25' is positioned in between the supporting EAP layer 25 and the flexible membrane 120. The supporting EAP layer 25 is actuated through the electrically conductive path 40 including the deformable switch 30, which enables the electrically conductive path 40 at increasing blood pressure of the patient such that at systolic blood pressures the supporting EAP layer 25 is actuated and generate a supporting force to the actuator 25' such that the shape of the balloon is better maintained throughout the cardiac cycle of the patient. As will be understood from the foregoing, the deformable switch 30 alternatively may have a variable impedance resistance such that the degree of actuation of the supporting EAP layer 25 increases with increasing blood pressure, such that the overall shape of the balloon can be even better maintained throughout the cardiac cycle.

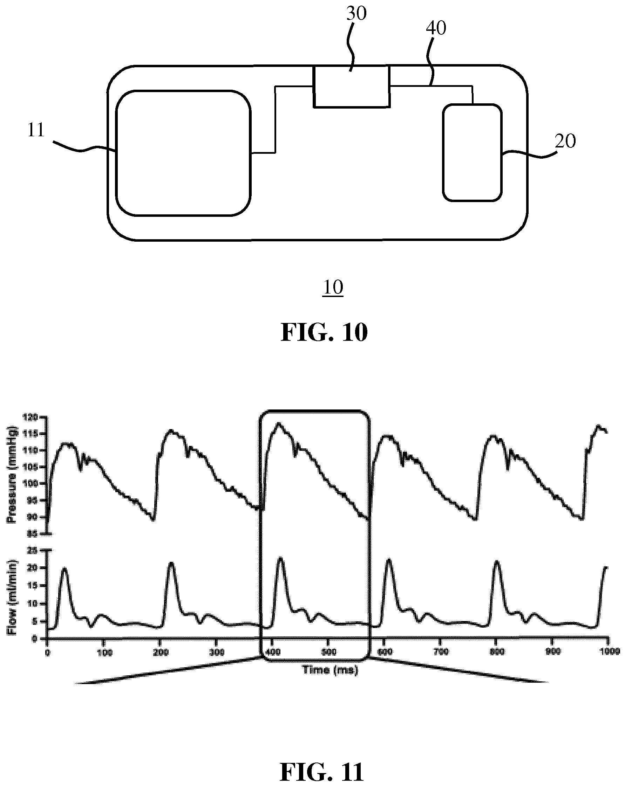

In the foregoing embodiments, the invasive medical device 10 has been described as a device that has a device portion 15 for inserting into the vascular system of the patient, e.g. a catheter or the like. However, embodiments of the present invention are not limited to such insertable medical devices. FIG. 10 schematically depicts an implantable medical device 10 according to an embodiment of the present invention in which a functional module 20, e.g. a sensor or actuator, is connected to a battery 11 through the electrically conductive path 40 including an embodiment of the deformable switch 30. The deformable switch 30 typically has a surface area in contact with the patient's blood, which surface area typically forms part of the housing of the implantable medical device 10 in which the various components of the device are housed.

In this embodiment, the deformable switch 30 is typically arranged to temporarily disconnect the functional module 20 from the battery 11, e.g. during parts of the cardiac cycle in which the functional module 20 cannot perform a meaningful task, such as a sensing task, a drug delivery task, and so on, such that operation of the functional module 20 during these parts of the cardiac cycle is avoided. This therefore increases the lifetime of the battery 11 and of the implantable medical device 10 as a consequence due to the fact that the functional module 20 does not consume power during these parts of the cardiac cycle.

FIG. 11 shows a graph depicting blood pressure (top graph) and resulting blood flow velocity (bottom graph) during multiple cardiac cycles, where it can be seen that the blood flow velocity shows strong variation throughout the cardiac cycle. More specifically, during systolic blood pressure the blood flow velocity through the vascular system of the patient is typically substantially higher than during diastolic blood pressure.

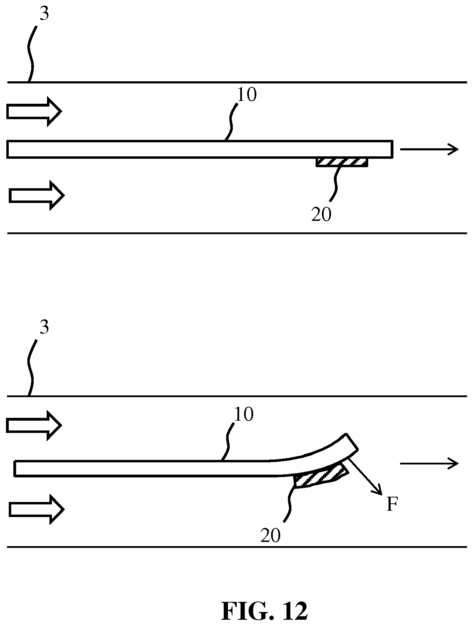

This has implications when inserting an invasive medical device 10 having a deformable device portion 15 into a blood vessel 3 of a patient as schematically depicted in FIG. 12. As is well-known per se, such a deformable device portion 15 maybe deformed in order to steer the invasive medical device 10, e.g. a catheter or the like, into a desired direction. The deformable device portion 15 may comprise a stiff region adjacent to a more flexible region, with the more flexible region carrying an actuator 20 such as an EAP actuator. In the off state of the actuator 20 as shown in the top pane of FIG. 12, the invasive medical device 10 may adopt a substantially straight configuration such that the device is more or less aligned with the direction of the blood flow as indicated by the block arrows.

However, upon actuation of the actuator 20 as shown in the bottom pane of FIG. 12, a tip of the invasive medical device 10 becomes deformed (bent). In this scenario, the blood flow will push against this deformed portion of the invasive medical device 10, thereby creating a force F counteracting the deformation force generated by the actuator 20. As will be understood from the foregoing, the magnitude of the counterforce F is a function of the blood flow velocity through the blood vessel 3 such that at higher blood pressure a larger counterforce F acts upon the deformed portion of the invasive medical device 10 such that when the actuator 20 is provided with a constant power supply, the degree of deformation of the deformable device portion 15 will be larger at lower blood pressures, e.g. diastolic blood pressure. This may lead to overbending of the deformable device portion 15, which may damage the invasive medical device 10 and/or cause damage to the blood vessel 3 of the patient.

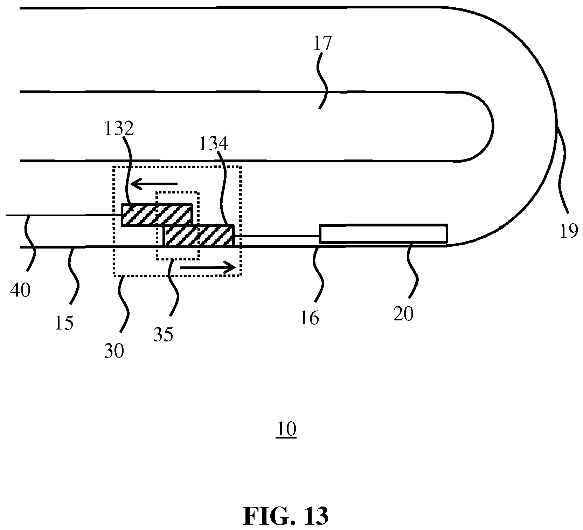

To address this problem, the invasive medical device 10 may comprise a strain-sensitive deformable switch 30 in or on the external surface 16 of the deformable device portion 15 as schematically depicted in FIG. 13. The strain-sensitive deformable switch 30 comprises a pair of electrodes 132, 134 that are slidably mounted relative to each other and are connected to opposing portions of the electrically conductive path 40. The pair of electrodes 132, 134 have a region or area of overlap 35 that varies as a function of the degree of deformation of the deformable device portion 15. With increased strain resulting from an increased degree of deformation of the deformable device portion 15, the pair of electrodes 132, 134 are pulled apart thereby reducing the area of overlap 35 between the pair of electrodes 132, 134 and increasing the resistance of the strain-sensitive deformable switch 30 such that at higher degree of deformation of the deformable device portion 15 a smaller actuation voltage is provided to the (EAP) actuator due to the higher voltage drop across the strain-sensitive deformable switch 30, thereby reducing the degree of actuation of the actuator 20 and protecting the deformable device portion 15 from overbending. In an embodiment, at a critical degree of bending of the deformable device portion 15 of the area of overlap 35 between the electrodes 132, 134 is zero, i.e. the electrically conductive path 40 is disrupted to prevent such overbending.

In this manner, the deformable device portion 15 of the invasive medical device 10, e.g. the deformable tip of a catheter, is kept stable in a dynamic blood pressure environment as fluctuations in the blood flow velocity resulting from the fluctuations in the patient's blood pressure are compensated for by the strain-sensitive deformable switch 30. Specifically, at lower blood pressure the increased tendency of the deformable device portion 15 to bend is compensated by the increased resistance of the strain-sensitive deformable switch 30, whereas at higher blood pressure the reduced tendency of the deformable device portion 15 to bend resulting from the increased counterforce F is compensated for by a reduced resistance of the strain-sensitive deformable switch 30 and a higher actuation voltage to the actuator 20.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. An invasive medical device (10) comprising:
a device portion (15) for inserting into a patient, said device portion including a functional module (20);
an electrically conductive path (40) extending through the invasive medical device and connecting to the functional module; and
a deformable switch (30) within the device portion arranged to alter the electrical impedance of said conductive path as a function of a degree of deformation of the switch, said deformation being caused by a blood parameter of the patient's blood that varies during the cardiac cycle of said patient.

2. The invasive medical device (10) of claim 1, wherein the functional module (20) comprises an electroactive polymer (25) and the electrically conductive path (40) is arranged to supply an actuation signal to the electroactive polymer.

3. The invasive medical device (10) of claim 1 or 2, wherein the blood parameter is blood pressure.

4. The invasive medical device (10) of claim 3, wherein the deformable switch (30) comprises a flexible membrane (131) in or on an external surface (16) of the device portion (15), said flexible membrane extending over a cavity (130) filled with a compressible medium, said cavity comprising a cavity floor (133) facing an inner surface of the flexible membrane, wherein said inner surface carries a first electrode arrangement and the cavity floor carries a second electrode arrangement facing the first electrode arrangement.

5. The invasive medical device (10) of claim 3, wherein the deformable switch (30) comprises a pressure-sensitive material portion in an external surface of the device portion (15), said pressure-sensitive material portion extending between opposing portions (40a, 40b) of the electrically conductive path (40) and comprising a plurality of electrically conductive particles (231) suspended in an electrically insulating matrix (230).

6. The invasive medical device (10) of claim 4, wherein the electrically conductive path (40) is disrupted when the first electrode arrangement is spatially separated from the second electrode arrangement.

7. The invasive medical device (10) of claim 6, wherein the first electrode arrangement comprises a first electrode (132) electrically connected to a first portion (40b) of the electrically conductive path (40) and the second electrode arrangement comprises a second electrode (134) electrically connected to a second portion (40b) of the electrically conductive path opposite said first portion.

8. The invasive medical device (10) of claim 6, wherein the first electrode arrangement comprises a first electrode (132) and the second electrode arrangement comprises a pair of spatially separated further electrodes (134a, 134b) electrically connected to opposing portions (40a, 40b) of the electrically conductive path (40), wherein the first electrode is arranged to bridge the spatially separated further electrodes when contacting the spatially separated further electrodes.

9. The invasive medical device (10) of any of claims 6-8, wherein the functional module (20) forms part of a functional module arrangement implementing a plurality of functions including a first function enabled by the electrically conductive path (40) and a second function enabled by a further electrically conductive path (40') extending through the invasive medical device and connecting to the functional module arrangement, and wherein the deformable switch (30) is arranged to disrupt the further electrically conductive path and enable the electrically conductive path at a first blood pressure of the patient and to disrupt the electrically conductive path and enable the further electrically conductive path at a second blood pressure of the patient different to said first blood pressure.

10. The invasive medical device (10) of any of claims 2-9, wherein the functional module (20) comprises a flexible module membrane (120) for providing a balloon function, and wherein the electroactive polymer (25) is arranged along an inner surface of the flexible module membrane.

11. The invasive medical device (10) of claim 10, further comprising a further electroactive polymer (25') in between the flexible module membrane (120) and the electroactive polymer (25) arranged to control the shape of the flexible module membrane, and wherein the electroactive polymer is arranged to support the further electroactive polymer as a function of the blood pressure of the patient.

12. The invasive medical device (10) of claim 2, wherein the device portion (15) is deformable and the electroactive polymer (25) is arranged to deform said device portion, and wherein the deformable switch (30) is arranged to alter the electrical impedance of said conductive path (40) with increasing deformation of the device portion.

13. The invasive medical device (10) of claim 12, wherein the deformable switch (30) comprises a pair of conductive portions (132, 134) connected to opposing portions of the electrically conductive path (40), wherein the conductive portions are slidably arranged relative to each other such that a contact area (35) between the conductive portions is altered with increasing deformation of the device portion (15).

14. The invasive medical device (10) of any of claims 1-13, wherein the invasive medical device is an implantable medical device comprising a battery (11), wherein the electrically conductive path (40) extends between the battery and the functional module (20).

15. The invasive medical device (10) of any of claims 1-14, wherein the deformable switch (30) is integral to the functional module (20).
